# EUROPEAN PATENT SPECIFICATION

(11) **EP 3 735 261 B1**
(45) Date of publication and mention of the grant of the patent: **15.05.2024**
(21) Application number: 18867689.4
(22) Date of filing: 11.10.2018
(51) Int. Cl.: A61K 38/18, A61P 35/00

(54) **GROWTH FACTOR TGFBETA1 OR TGFBETA2 OR TGFBETA3 FOR THE USE IN ANTICANCER THERAPY FOR PATIENTS WITH TUMORS, IN WHICH AT LEAST PART OF CELLS EXPRESS EGFRVIII**
WACHSTUMSFAKTOR TGFBETA1 ODER TGFBETA2 ODER TGFBETA3 FÜR DIE VERWENDUNG IN DER ANTIKREBSTHERAPIE FÜR PATIENTEN MIT TUMOREN, IN DENEN MINDESTENS EIN TEIL DER ZELLEN EGFRVIII EXPRIMIEREN
FACTEUR DE CROISSANCE TGFBETA1 OU TGFBETA2 OU TGFBETA3 POUR SON UTILISATION EN THÉRAPIE ANTICANCÉREUSE POUR PATIENTS AYANT DES TUMEURS OÙ UNE PARTIE AU MOINS DES CELLULES EXPRIMENT L'EGFR VIII

(30) Priority: 19.10.2017 PL 42320317; 09.10.2018 PL 42735818
(43) Date of publication of application: 11.11.2020
(73) Proprietor: Celther Polska Sp. z o.o., 95-050 Konstantynow Lodzki (PL); Personather Sp. z o.o., 95-050 Konstantynow Lodzki (PL); Rieske, Piotr, 91-013 Lodz (PL); Stoczynska-Fidelus, Ewelina, 96-124 Makow (PL); Piaskowski, Sylwester, 95-050 Konstantynow Lodzki (PL); Wyrzykowski, Andrzej Szczepan, 03-252 Warszawa (PL)
(72) Inventor: RIESKE, Piotr, 91-013 Lodz (PL); STOCZYNSKA-FIDELUS, Ewelina, 96-124 Makow (PL); WYRZYKOWSKI, Andrzej Szczepan, 03-252 Warszawa (PL)
(74) Representative: Kondrat, Mariusz
(86) International application number: PCT/PL2018/000097
(87) International publication number: WO 2019/078745

(56) References cited:
- XU ZHANG ET AL: "Increased transforming growth factor-2 in epidermal growth factor receptor variant III-positive glioblastoma", JOURNAL OF CLINICAL NEUROSCIENCE, CHURCHILL LIVINGSTONE, GB, vol. 18, no. 6, 11 September 2010 (2010-09-11), pages 821-826, XP028206563, ISSN: 0967-5868, DOI: 10.1016/J.JOCN.2010.09.024 [retrieved on 2010-12-16]
- DEL VECCHIO C A ET AL: "EGFRvIII gene rearrangement is an early event in glioblastoma tumorigenesis and expression defines a hierarchy modulated by epigenetic mechanisms", ONCOGENE, vol. 32, no. 21, 16 July 2012 (2012-07-16) , pages 2670-2681, XP055892220, London ISSN: 0950-9232, DOI: 10.1038/onc.2012.280 Retrieved from the Internet: URL:http://www.nature.com/articles/onc2012 280>
- NANA, A. W. et al.: "Overview of Transforming Growth Factor beta Superfamily Involvement in Glioblastoma Initiation and Progression", Asian Pacific Journal of Cancer Prevention, vol. 16 2015, page 6816, XP055597521, Retrieved from the Internet: URL:http://dx.doi.org/107314/APJCP.2015.16 .16.6813
- SAMPSON, J. H. et al.: "Tumor-specific Immunotherapy Targeting the EGFRvIII Mutation in Patients with Malignant Glioma", Seminars in Immunology, vol. 20, no. 5, October 2008 (2008-10), pages 267-275, XP025646566, DOI: doi:10.1016/j.smim.2008.04.001
- GALLO-OLLER, G. et al.: "P144, a Transforming Growth Factor beta inhibitor peptide, generates antitumoral effects and modifies SMAD7 and SKI levels in human glioblastoma cell lines", Cancer Letters, vol. 381, 2016, pages 67-75, XP029715488, DOI: doi:10.1016/j.canlet.2016.07.029
- CHEN, W. et al.: "TGF- beta Regulates Survivin to Affect Cell Cycle and the Expression of EGFR and MMP9 in Glioblastoma", Molecular Neurobiology, vol. 53, 2016, pages 1648-1653, XP036236800,

## Description

The following invention relates to the use of TGFβ1 (transforming growth factor type beta 1) or TGFβ2 (transforming growth factor type beta 2) or TGFβ3 (transforming growth factor type beta 3) in the treatment of patients with neoplastic lesions characterized by the presence of tumor cells with a specific phenotype, namely the presence of a mutated EGF receptor, i.e. EGFR^{vIII},

### Description of the state of the art

Mutation leading to the formation of so-called EGFR^{vIII} can be observed in many cancers. Currently, it is assumed that antitumor activity concerning EGFR^{vIII}-positive tumor cells may involve induction of their cell death *via* inhibition of kinase activity of this receptor. Commonly available forms of therapy, including chemotherapy, radiotherapy and surgery were also applied. Attempts to use immunological therapies have also been made, however, appeared to be unsuccessful.

So far, no one has suggested to use TGFβ1 or TGFP2 or TGFβ3 in the treatment of patients with tumors, in which cells express EGFR^{vIII}.

EGFR^{vIII} results from the deletion of a fragment of EGFR containing exons 2-7 [Wikstrand 1998]. This mutant does not occur in normal cells. Due to the fact that EGFR^{vIII} expression is present in many cancer types, there is a huge need for therapy eliminating cancer cells expressing EGFR^{vIII}.

Cancer cells expressing EGFR^{vIII} are characterized by high drug resistance to small active molecules- [Liu 2013, Learn 2004, Schulte 2013, Ji 2006,Zanca 2017] and immune-based therapies [Brand 2011, Yang 2008, Patel 2007].

EGFR^{vIII}-positive cells may play a role of cancer stem cells, or, possibly, EGFR^{vIII}-negative cells may depend on EGFR^{vIII}-positive cells [Zanca 2017, P 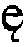 ciak 2017].

There is a very large demand for a drug that is specific for cells expressing EGFR^{vIII}. At the moment, there are no substances that lead to death of tumor cells expressing EGFR^{vIII}, but are simultaneously relatively harmless to normal cells, on the market. Drugs used currently are associated with very serious side effects, as targets of these drugs can be usually found in both, normal and cancer cells [Dudek 2006, Perez-Soler 2004, Petty 2003, Nguyen 2009].

Tumor cells with high EGFR^{vIII} expression level are mostly glioblastoma cells [P 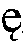 eciak 2017]. There are many other cancer types characterized by the presence of EGFR^{vIII}-positive cells.

However, in case of glioblastoma, the median survival rate is only 15-18 months. The most optimistic data indicate that only a few percent of patients diagnosed with glioblastoma in the US will survive more than five years from the diagnosis. For comparison, only 10% of women diagnosed with breast cancer in the US will not survive five years from the diagnosis. In case of glioblastoma, the longest survival can be achieved for patients treated with the combination of radiotherapy, temozolomide (chemotherapy) and surgery. The therapy is usually accompanied by extreme deterioration of patients and dramatic personality changes.

Almost half of patients diagnosed with glioblastoma are elder people (approximately 70-year-olds), who very badly tolerate the currently used types of therapy. There is no consensus as to what therapeutic standards should be applied in case of such patients. Median survival for these patients is about ten months. There is a lack of therapy leading to death of glioblastoma cancer cells and harmless to normal cells that would improve the situation of patients with this tumor type. Such anti-cancer therapies have not been discovered yet or constitute an absolute rarity. Attempts have been made to use immunological therapies in case of glioblastoma patients, as these could meet the criterion of obvious harmlessness, however, these were unsuccessful [Hopper-Borge 2009, Grisanti 2008, Rades 2010].

TGFβ1, TGFβ2, TGFβ3 are generally known to have a different effect on cancer cells than that described in this application. So far, attempts have been made to use TGFβ receptor inhibitors in oncological therapy. There are no known attempts to use TGFβ1, TGFβ2, TGFβ3 as an anti-cancer agents, especially in the treatment of cancers with EGFR^{vIII}-positive cells.

It is well known that TGFβ1, TGFβ2, TGFβ3 cannot be as harmful to normal cells as, e.g., erlotinib, afatinib or temozolomide are. TGFβ is a substance produced in the human body under physiological conditions. Its harmfulness is, therefore, relatively negligible.

### Detailed description of the subject matter of the invention

The subject matter of the invention is TGFβ1, TGFβ2, TGFβ3 (transforming growth factor beta 1 or transforming growth factor beta 2 or transforming growth factor beta 3), described in the HPRD protein database under the identifier number 01821 (TGFβ1), 01828 (TGFP2) and 01829 (TGFP3), for the use in treatment of patients with neoplastic lesions in which tumor cells express EGFR^{vIII} (characterized by the absence of the region encoded by exons 2-7). TGFβ1 was provided by Merck EMD Millipore, TGFP2 was provided by Sigma-Aldrich, TGFβ3 was provided by Gibco^{™} (Thermo Fisher Scientific).

The subfamily of TGFβ factors for which the receptor is TGFβR consists of three factors: β1, β2 and β3. Each of them in an active form consists of 112 amino acids.

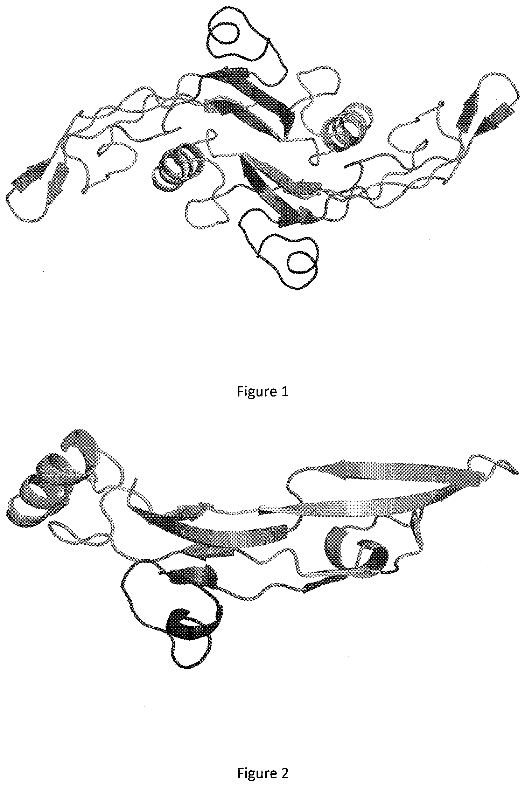

TGFβ1 initially consists of 390 amino acids, from which an active sequence of 112 amino acids is obtained. These are amino acids 279 to 390 of the total amino acid sequence of TGFβ1. It was the active form of TGFβ1 that was included in the analyzes. It should be remembered that the TGFβ1 protein is glycosylated at positions 82, 136 and 176. Moreover, there are disulfide bridges within the structure (Fig. 1).

AL DTNYCFSSTE KNCCVRQLYIDFRKDLGWKW IHEPKGYHAN FCLGPCPYIW
SLDTQYSKVL ALYNQHNPGASAAPCCVPQA LEPLPIVYYV GRKPKVEQLS
NMIVRSCKCS

**Formula 1**. The amino acid sequence of the active part of the growth factor, herein after referred to as TGFβ1.

TGFP2 initially consists of 414 amino acids, from which an active sequence of 112 amino acids is obtained. These are amino acids 303 to 414 of the total amino acid sequence of TGFP2. It was the active form of TGFβ2 that was included in the analyzes. It should be remembered that the TGFβ2 protein is glycosylated at positions 72, 140 and 241. Moreover, there are disulfide bridges within the structure (Fig. 2).

ALDAAYCF RNVQDNCCLR PLYIDFKRDL GWKWIHEPKG YNANFCAGAC
PYLWSSDTQH SRVLSLYNTI NPEASASPCC VSQDLEPLTI LYYIGKTPKI
EQLSNMIVKS CKCS

**Formula 2**. The amino acid sequence of the active part of the growth factor, herein after referred to as TGFβ2.

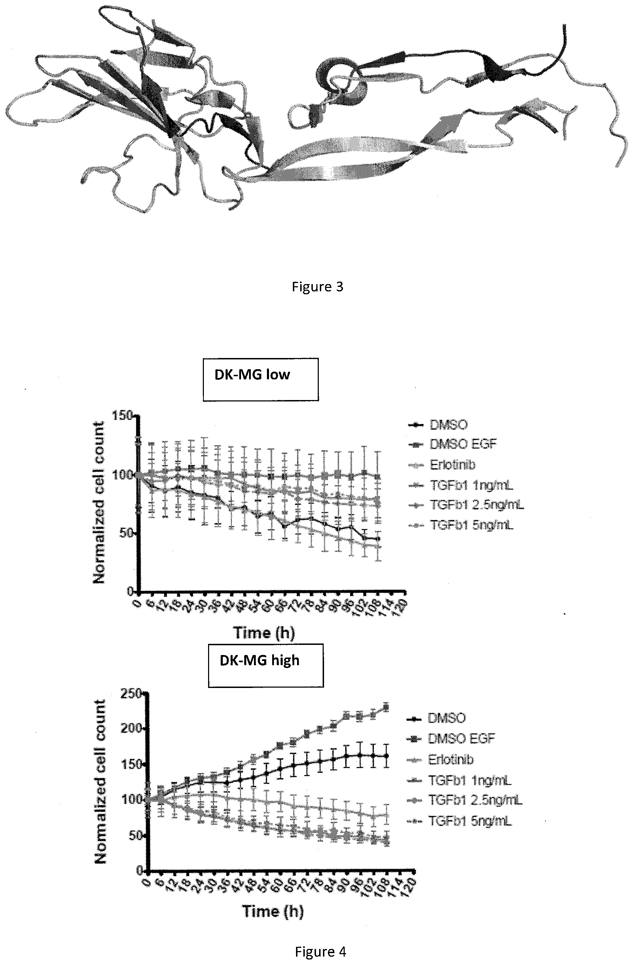

TGFβ3 initially consists of 412 amino acids, from which as well as other factors from the TGFβ subfamily, an active sequence of 112 amino acids is obtained. These are amino acids 301 to 412 of the total amino acid sequence of TGFβ3. It was the active form of TGFβ3 that was included in the analyzes. It should be remembered that the TGFβ3 protein is glycosylated at positions 74, 135 and 142. Moreover, there are disulfide bridges within the structure (Fig. 3).

**Formula 3.** The amino acid sequence of the active part of the growth factor, herein after referred to as TGFβ3.

The results of *in vitro* and *in vivo* analyses (on an animal model) are included within the examples. Analyses were conducted using a commercially available DK-MG cell line, from which two sublines were derived. The first subline, DK-MG high, demonstrated from 40% to 70% of EGFR^{vII1}-positive cells. The second subline, DK-MG low, showed no more than 5% of EGFR^{vIII}-positive cells. These sublines are described in the article by Stec *et al.* [Oncotarget 2017]. TGFβ1 has also been tested on glioblastoma cells that do not express EGFR^{vIII}. There was no apparent effect of TGFβ1 on EGFR^{vIII}-negative cells of cell line other than DK-MG.

The line of immortalized murine fibroblasts - Balb/3T3, as well as normal human fibroblasts, human pericytes and human induced neural stem cells were used to evaluate the toxicity of tested compound to normal cells.

Conducted analyses demonstrated extreme toxicity of TGFβ1 or TGFβ2 or TGFβ3 to tumor cells expressing EGFR^{vIII} and lack of toxic effect (TGFβ1), possibly, toxicity to a statistically insignificant degree (TGFP2 or TGFβ3) on the various normal cell lines mentioned above. In case of normal cells, a beneficial effect of TGFβ1 on their proliferation was observed. A similar beneficial effect on proliferation was also observed in case of U87MG glioblastoma cell line. Normal cells were included in the analyses, despite the fact that it is known that TGFP cannot be as toxic to these cells as small-molecule therapeutic substances (e.g. erlotinib or temozolomide). The TGFβ1 obtained from Merck EMD Millipore (cat. No. GF111), TGFβ2 (cat. No. SRP3170-5UG; Sigma-Aldrich) and TGFβ3 (cat. No. PHG9305; Gibco^{™}; Thermo Fisher Scientific) were used in the analyses. The active forms of TGFβ consisting of 112 amino acids were used.

Animal model studies were performed on SCID mice model (NOD.CB17-Prkdcscid/J), using DK-MG high subline and showed the reduction in tumor size (volume) when compared to the negative control, without affecting the general condition of the animal.

### Literature

Brand TM, Iida M, Wheeler DL. Molecular mechanisms of resistance to the EGFR monoclonal antibody cetuximab. Cancer BiolTher. 2011 May 1;11(9):777-92.
Dudek AZ,Kmak KL, Koopmeiners J, Keshtgarpour M. Skin rash and bronchoalveolar histology correlates with clinical benefit in patients treated with gefitinib as a therapy for previously treated advanced or metastatic non-small cell lung cancer. Lung Cancer. 2006 Jan;51(1):89-96.
Emrich JG, Brady LW, Quang TS, et al. Radioiodinated (I-125) monoclonal antibody 425 in the treatment of high grade glioma patients: ten-year synopsis of a novel treatment. Am J ClinOncol 2002;25:541-6.
**GrisantiS**,AmorosoV,BuglioneM,RosatiA,GattaR,PizzocaroC,FerrariVD,MariniG.Cetux imabinthetreatmentofmetastaticmucoepidermoidcarcinomaofthesalivaryglands:acaserepor tandreviewofliterature.JMedCaseRep.2008Sep30;2:320.
Han J, Alvarez-Breckenridge CA, Wang Q, Yu J. TGF-β signaling and its targeting for glioma treatment. Am J Cancer Res. 2015; 5(3): 945-955.
Hopper-BorgeEA, NastoRE, RatushnyV, WeinerLM, GolemisEA, AstsaturovI. Mechanisms of tumor resistance to EGFR-targeted therapies. ExpertOpinTherTargets. 2009 Mar;13(3):339-62.
Ji H, Li D, Chen L, Shimamura T, Kobayashi S, McNamara K, Mahmood U, Mitchell A, Sun Y, Al-Hashem R, Chirieac LR, Padera R, Bronson RT, Kim W, Jänne PA, Shapiro GI, Tenen D, Johnson BE, Weissleder R, Sharpless NE, Wong KK. The impact of human EGFR kinase domain mutations on lung tumorigenesis and in vivo sensitivity to EGFR-targeted therapies. Cancer Cell. 2006 Jun;9(6):485-95.
Learn CA, Hartzell TL, Wikstrand CJ, Archer GE, Rich JN, Friedman AH, Friedman HS, Bigner DD, Sampson JH. Resistance to tyrosine kinase inhibition by mutant epidermal growth factor receptor variant III contributes to the neoplastic phenotype of glioblastoma multiforme. Clin Cancer Res. 2004;10:3216-3224.
Liu XJ, Wu WT, Wu WH, Yin F, Ma SH, Qin JZ, Liu XX, Liu YN, Zhang XY, Li P, Han S, Liu KY, Zhang JM, He QH, Shen L. A minority subpopulation of CD133(+) /EGFRvIII(+) /EGFR(-) cells acquires sternness and contributes to gefitinib resistance. CNS NeurosciTher. 2013 Jul;19(7):494-502.
Nguyen A, Hoang V, Laquer V, Kelly KM (December 2009). "Angiogenesis in cutaneous disease: part I". J. Am. Acad. Dermatol. 61 (6): 921-42; quiz 943-4.
Patel D, Lahiji A, Patel S, Franklin M, Jimenez X, Hicklin DJ, Kang X. Monoclonal antibody cetuximab binds to and down-regulates constitutively activated epidermal growth factor receptor vIII on the cell surface. Anticancer Res. 2007 Sep-Oct;27(5A):3355-66.
Peciak J,Stec WJ, Treda C, Ksiazkiewicz M, Janik K, Popeda M, Smolarz M, Rosiak K, Hulas-Bigoszewska K, Och W, Rieske P, Stoczynska-Fidelus E.Low Incidence along with Low mRNA Levels of EGFRvIII in Prostate and Colorectal Cancers Compared to Glioblastoma. J Cancer. 2017 Jan 1;8(1):146-151. doi: 10.7150/jca.16108. eCollection 2017.
Román Pérez-Soler, M.D., et al. (2004). "Selected Highlights". Lung Cancer Frontiers 22 (16): 3238-3247.
Rusch V, Baselga J, Cordon-Cardo C iwsp. Differential expression of the epidermal growth factor receptor and its ligands in primary non-small cell lung cancers and adjacent benign lung. Cancer Res 1993; 53: 2379-85.
Stec WJ, Rosiak K, Siejka P, Peciak J, Popeda M, Banaszczyk M, Pawlowska R, Treda C, Hulas-Bigoszewska K, Piaskowski S, Stoczynska-Fidelus E, Rieske P.Cell line with endogenous EGFRvIII expression is a suitable model for research and drug development purposes.Oncotarget. 2016 May 31;7(22):31907-25. doi: 10.18632/oncotarget.8201.
Schulte A, Liffers K, Katliagen A, Riethdorf S, Zapf S, Merlo A, Kolbe K, Westphal M, Lamszus K.Erlotinib resistance in EGFR-amplified glioblastoma cells is associated with upregulation of EGFRvIII and PI3Kp110δ. Neuro Oncol. 2013 Oct;15(10):1289-301.
Thomas L. Petty, M.D. (2003). "Determinants of Tumor Response and Survival With Erlotinib in Patients With Non-Small-Cell Lung Cancer". Journal of Clinical Oncology 1 (17): 3-4.
Wikstrand CJ, McLendon RE, Friedman AH, et al. Cell surface localization and density of the tumor-associated variant of the epidermal growth factor receptor, EGFRvIII. Cancer Res 1997; 57: 4130-40.
Wikstrand CJ, Reist CJ, Archer GE, Zalutsky MR, Bigner DD. The class III variant of the epidermal growth factor receptor (EGFRvIII): characterization and utilization as an immunotherapeutic target J Neurovirol. 1998; 4(2):148-58.
Yang CH, Yu CJ, Shih JY, Chang YC, Hu FC, Tsai MC, Chen KY, Lin ZZ, Huang CJ, Shun CT, Huang CL, Bean J, Cheng AL, Pao W, Yang PC. Specific EGFR mutations predict treatment outcome of stage IIIB/IV patients with chemotherapy-naive non-small-cell lung cancer receiving first-line gefitinib monotherapy. J ClinOncol. 2008 Jun 1;26(16):2745-53.
Zanca C, Villa GR, Benitez JA, Thorne AH, Koga T, D'Antonio M, Ikegami S, Ma J, Boyer AD, Banisadr A, Jameson NM, Parisian AD, Eliseeva OV, Barnabe GF, Liu F, Wu S, Yang H, Wykosky J, Frazer KA, Verkhusha VV, Isaguliants MG, Weiss WA, Gahman TC, Shiau AK, Chen CC, Mischel PS, Cavenee WK, Furnari FBGenes Dev. 2017 Jul 19. doi: 10.1101/gad.300079.117.

### Examples

### Example 1. Analysis of the impact of tested compound on DK-MG cell line (DK-MG high and low sublines) based on imaging assays (BioStation CT).

The example illustrates the effect of TGFβ1 on survival and proliferation of DK-MG cells (two sublines). For this purpose, the study was conducted using cells of two DK-MG sublines - one showing no less than 40% of EGFR^{vIII}-positive cells (DK-MG high) and second one exhibiting no more than 5% of EGFR^{vIII}-positive cells (DK-MG low). DK-MG low and high sublines are described in the article by Stec et al. Oncotarget 2017.

### Day 1

1. Rinse DK-MG high/low cells at 90% confluence with phosphate-buffered saline (PBS) w/o Mg²⁺ i Ca²⁺. Remove PBS.
2. Detach cells from the culture vessel by adding Trypsin-EDTA (0.05%, 1x) and incubate for 1-3 min at 37°C.
3. Transfer cells to 15 ml conical tube, centrifuge (200xg, 4 min) and suspend in RPMI 1640 culture medium supplemented with 10% fetal bovine serum and antibiotics.
4. Count cells.
5. Add 5×10⁴ cells per well of 6-well culture plate for adherent culture.
6. Supply with aforementioned cell culture medium and leave for about 12 hours at 37°C.

### Day 2

1. Rinse 2-3 times with PBS w/o Mg²⁺ i Ca²⁺. Remove PBS precisely each time.
2. Add 2.5 ml of RPMI 1640 culture medium with antibiotics and w/o serum with appropriate molecules (DMSO; maximum volume of tested compounds); alone or in combination with EGF (20 ng/ml (Sigma, Cat. no. E9644)), erlotinib (1-25 µM Molecula; Cat. No. 89983631), TGFβ1 (1 to 25 ng/ml Cat. no.. GF111 Merck EMD Millipore).
3. Place cell culture vessel into BioStation CT. Define cell culture plate and each well
4. Perform automatic microscopic observation at 3-hour intervals.

### The following days

1. Change culture media each 24 hours. Do not forget to add DMSO/EGF/erlotinib/TGFβ individually or in combinations to proper culture vessels.
2. Place cell culture vessels into BioStation CT.
3. Perform automatic microscopic observation at 3-hour intervals. BioStation CT software allows automatic cell counting and assessment of surface area occupied by these cells.
4. Analogous observations, however, with less accuracy can be performed using JULI microscope or an ordinary light microscope. However, it should be emphasized that BioStation CT enables the preservation of environmental conditions during the experiment as well as the observation of many vessels at the same time.

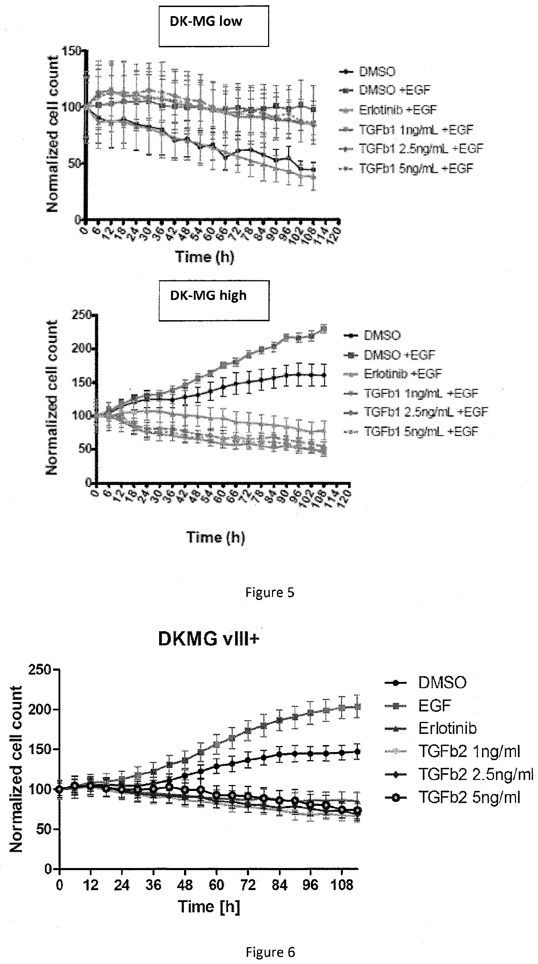

**Results:** TGFβ1 is effective against DK-MG tumor cells expressing EGFR^{vIII} and their dependent cells - It inhibits their proliferation and induces cell death (Fig. 4, Fig. 5).

### Example 2. Toxicity analysis of TGFβ2 on DK-MG high cells using BioStation CT device.

The example illustrates the effect of TGFβ2 on the survival and proliferation of DK-MG high cell lines. For this purpose, experiment was performed on cell lines displaying not less than 40% of EGFRvIII positive cells (DK-MG high). This cell subline was described in the article Stec et al. Onotarget 2017.

### Day 1

1. Rinse DK-MG high cells at 90% confluence with phosphate-buffered saline (PBS) w/o Mg²⁺ i Ca²⁺. Remove PBS.
2. Detach cells from the culture vessel by adding Trypsin-EDTA (0.05%, 1x) and incubate for 1-3 min at 37°C.
3. Transfer cells to 15 ml conical tube, centrifuge (200xg, 4 min) and suspend in RPMI 1640 culture medium supplemented with 10% fetal bovine serum and antibiotics.
4. Count cells.
5. Add 4×10⁴ cells per well of 6-well culture plate for adherent culture.
6. Supply with mentioned cell culture medium and leave for about 12 hours at 37°C.

### Day 2

1. Rinse cells 2-3 times with PBS w/o Mg²⁺ i Ca²⁺. Remove PBS precisely each time.
2. Add 2 ml of RPMI 1640 culture medium with antibiotics and w/o serum and leave for about 12 hours at 37°C.

### Day 3

1. Rinse cells 2-3 times with PBS w/o Mg²⁺i Ca²⁺. Remove PBS precisely each time.
2. Add 2 ml of RPMI 1640 culture medium with antibiotics and w/o.
3. Do not forget to add (DMSO; max. volume of tested compounds); alone or in combination with EGF (20 ng/ml (Sigma, Cat. no. E9644)), erlotynib (10 µM Molecula; Cat. No. 89983631), TGFβ2 (1; 2,5; 5 ng/ml Cat. No. SRP3170-5UG; Sigma-Aldrich).
4. Place cell culture vessels into BioStation CT.
5. Perform automatic microscopic observation at 6-hour intervals. BioStation CT software allows automatic cell counting and assessment of surface area occupied by these cells.

### The following days

1. Change culture media each 24 hours. Do not forget to add DMSO/EGF/erlotinib/TGF02 individually or in combinations to proper culture vessels.
2. Place cell culture vessels into BioStation CT.
3. Perform automatic microscopic observation at 3-hour intervals. BioStation CT software allows automatic cell counting and assessment of surface area occupied by these cells.
4. Analogous observations, however, with less accuracy can be performed using JULI microscope or an ordinary light microscope. However, it should be emphasized that BioStation CT enables the preservation of environmental conditions during the experiment as well as the observation of many vessels at the same time.

**Results:** TGFβ2 is effective against DK-MG tumor cells expressing EGFR^{vIII} and their dependent cells - It inhibits their proliferation and induces cell death (Fig. 6). IC-50 value of TGFβ2 on this cell line fluctuated in the range of a few ng/ml.

### Example 3. Analysis of the effect of TGFβ2 on DK-MG low cell line based on imaging methods (BioStation CT)

The example illustrates the effect of TGFβ2 on survival and proliferation of DK-MG low cells. For this purpose, the study was conducted using cells exhibiting no more than 5% of EGFR^{vIII}-positive cells (DK-MG low). DK-MG low was described in the article by Stec et al. Oncotarget 2017.

### Day 1

1. Rinse DK-MG low cells at 90% confluence with phosphate-buffered saline (PBS) w/o Mg²⁺ i Ca²⁺. Remove PBS.
2. Detach cells from the culture vessel by adding Trypsin-EDTA (0.05%, 1x) and incubate for 1-3 min at 37°C.
3. Transfer cells to 15 ml conical tube, centrifuge (200xg, 4 min) and suspend in RPMI 1640 culture medium supplemented with 10% fetal bovine serum and antibiotics.
4. Count cells.
5. Add 4×10⁴ cells per well of 6-well culture plate for adherent culture.
6. Supply with mentioned cell culture medium and leave for about 12 hours at 37°C.

### Day 2

1. Rinse 2-3 times with PBS w/o Mg²⁺ i Ca²⁺. Remove PBS precisely each time.
2. Add 2 ml of RPMI 1640 culture medium with antibiotics and w/o serum and leave for about 12 hours at 37°C.

### Day 3

1. Rinse cells 2-3 times with PBS w/o Mg²⁺ i Ca²⁺. Remove PBS precisely each time.
2. Add 2 ml of RPMI 1640 culture medium with antibiotics and w/o.
3. Do not forget to add (DMSO; max. volume of tested compounds); alone or in combination with EGF (20 ng/ml (Sigma, Cat. no. E9644)), erlotynib (10 µM Molecula; Cat. No. 89983631), TGFβ2 (1; 2,5; 5 ng/ml Cat. No. SRP3170-5UG; Sigma-Aldrich).
4. Place cell culture vessels into BioStation CT.
5. Perform automatic microscopic observation at 6-hour intervals. BioStation CT software allows automatic cell counting and assessment of surface area occupied by these cells.

### The following days

1. Change culture media each 24 hours. Do not forget to add DMSO/EGF/erlotinib/TGF02 individually or in combinations to proper culture vessels.
2. Place cell culture vessels into BioStation CT.
3. Perform automatic microscopic observation at 3-hour intervals. BioStation CT software allows automatic cell counting and assessment of surface area occupied by these cells.
4. Analogous observations, however, with less accuracy can be performed using JULI microscope or an ordinary light microscope. However, it should be emphasized that BioStation CT enables the preservation of environmental conditions during the experiment as well as the observation of many vessels at the same time.

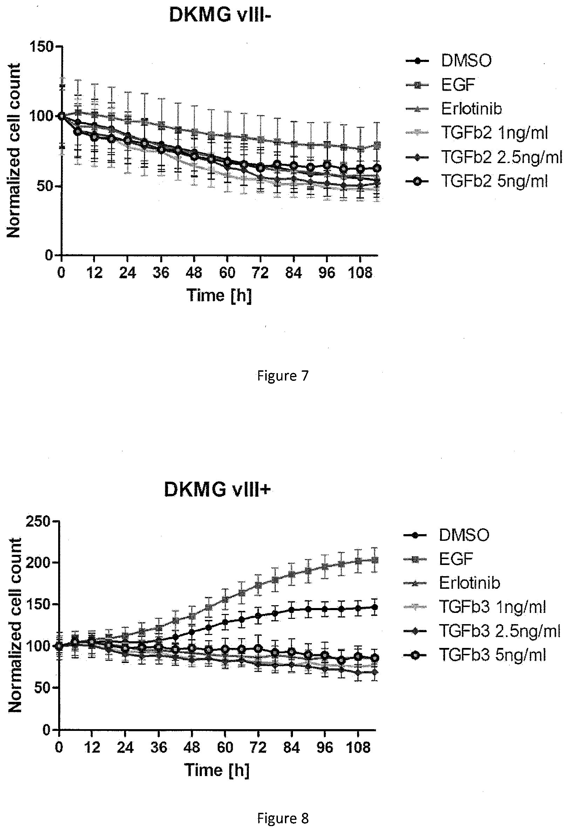

**Results:** TGFβ2 is ineffective against DK-MG tumor cells and without expressing EGFR^{vIII} dependent cells (Fig. 7). The presented result indicates the lack of toxicity of the TGFP2 molecule towards the DK-MG low cell line under the analyzed conditions.

### Example 4. Analysis of the effect of TGFβ3 on DK-MG high cell line based on imaging methods (BioStation CT)

The example illustrates the effect of TGFP3 on survival and proliferation of DK-MG high cells. For this purpose, the study was conducted using cells showing no less than 40% of EGFR^{vIII}-positive cells (DK-MG high). DK-MG high was described in the article by Stec et al. Oncotarget 2017.

### Day 1

1. Rinse DK-MG high cells at 90% confluence with phosphate-buffered saline (PBS) w/o Mg²⁺ i Ca²⁺. Remove PBS.
2. Detach cells from the culture vessel by adding Trypsin-EDTA (0.05%, 1x) and incubate for 1-3 min at 37°C.
3. Transfer cells to 15 ml conical tube, centrifuge (200xg, 4 min) and suspend in RPMI 1640 culture medium supplemented with 10% fetal bovine serum and antibiotics.
4. Count cells.
5. Add 4×10⁴ cells per well of 6-well culture plate for adherent culture.
6. Supply with mentioned cell culture medium and leave for about 12 hours at 37°C.

### Day 2

1. Rinse 2-3 times with PBS w/o Mg²⁺ i Ca²⁺. Remove PBS precisely each time.
2. Add 2 ml of RPMI 1640 culture medium with antibiotics and w/o serum and leave for about 12 hours at 37°C.

### Day 3

1. Rinse cells 2-3 times with PBS w/o Mg²⁺ i Ca²⁺. Remove PBS precisely each time.
2. Add 2 ml of RPMI 1640 culture medium with antibiotics and w/o.
3. Do not forget to add (DMSO; max. volume of tested compounds); alone or in combination with EGF (20 ng/ml (Sigma, Cat. no. E9644)), erlotynib (10 µM Molecula; Cat. No. 89983631), TGFβ3 (1; 2,5; 5 ng/ml Cat. No. PHG9305; Gibco^{™}; Thermo Fisher Scientific).
4. Place cell culture vessels into BioStation CT.
5. Perform automatic microscopic observation at 6-hour intervals. BioStation CT software allows automatic cell counting and assessment of surface area occupied by these cells.

### The following days

1. Change culture media each 24 hours. Do not forget to add DMSO/EGF/erlotinib/TGFβ3 individually or in combinations to proper culture vessels.
2. Place cell culture vessels into BioStation CT.
3. Perform automatic microscopic observation at 3-hour intervals. BioStation CT software allows automatic cell counting and assessment of surface area occupied by these cells.
4. Analogous observations, however, with less accuracy can be performed using JULI microscope or an ordinary light microscope. However, it should be emphasized that BioStation CT enables the preservation of environmental conditions during the experiment as well as the observation of many vessels at the same time.

**Results:** TGFβ3 is effective against DK-MG tumor cells expressing EGFR^{vIII} and dependent cells - inhibits their proliferation and leads to cell death. IC-50 value for TGFβ3 against these cells fluctuated in the range of a few ng/ml.

### Example 5. Analysis of the effect of TGFβ3 on DK-MG low cell line based on imaging methods (BioStation CT)

The example illustrates the effect of TGFP3 on survival and proliferation of DK-MG low cells. For this purpose, the study was conducted using cells exhibiting no more than 5% of EGFR^{vIII}-positive cells (DK-MG low). DK-MG low was described in the article by Stec et al. Oncotarget 2017.

### Day 1

1. Rinse DK-MG low cells at 90% confluence with phosphate-buffered saline (PBS) w/o Mg²⁺ i Ca²⁺. Remove PBS.
2. Detach cells from the culture vessel by adding Trypsin-EDTA (0.05%, 1x) and incubate for 1-3 min at 37°C.
3. Transfer cells to 15 ml conical tube, centrifuge (200xg, 4 min) and suspend in RPMI 1640 culture medium supplemented with 10% fetal bovine serum and antibiotics.
4. Count cells.
5. Add 4×10⁴ cells per well of 6-well culture plate for adherent culture.
6. Supply with mentioned cell culture medium and leave for about 12 hours at 37°C.

### Day 2

1. Rinse 2-3 times with PBS w/o Mg²⁺ i Ca²⁺. Remove PBS precisely each time.
2. Add 2 ml of RPMI 1640 culture medium with antibiotics and w/o serum and leave for about 12 hours at 37°C.

### Day 3

1. Rinse cells 2-3 times with PBS w/o Mg²⁺ i Ca²⁺. Remove PBS precisely each time.
2. Add 2 ml of RPMI 1640 culture medium with antibiotics and w/o serum.
3. Do not forget to add (DMSO; max. volume of tested compounds); alone or in combination with EGF (20 ng/ml (Sigma, Cat. no. E9644)), erlotynib (10 µM Molecula; Cat. No. 89983631), TGFβ3 (1; 2,5; 5 ng/ml, Cat. No. PHG9305; Gibco^{™}; Thermo Fisher Scientific).
4. Place cell culture vessels into BioStation CT.
5. Perform automatic microscopic observation at 6-hour intervals. BioStation CT software allows automatic cell counting and assessment of surface area occupied by these cells.

### The following days

1. Change culture media each 24 hours. Do not forget to add DMSO/EGF/erlotinib/TGFβ3 individually or in combinations to proper culture vessels.
2. Place cell culture vessels into BioStation CT.
3. Perform automatic microscopic observation at 3-hour intervals. BioStation CT software allows automatic cell counting and assessment of surface area occupied by these cells.
4. Analogous observations, however, with less accuracy can be performed using JULI microscope or an ordinary light microscope. However, it should be emphasized that

BioStation CT enables the preservation of environmental conditions during the experiment as well as the observation of many vessels at the same time.

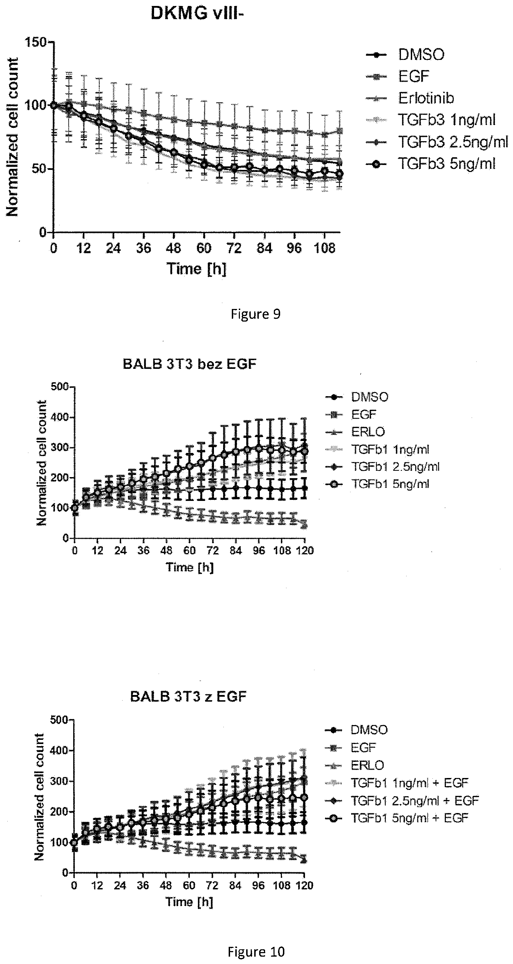

**Results:** TGFβ3 is ineffective against DK-MG tumor cells and without expressing EGFR^{vIII} dependent cells (Fig. 9). The presented result indicates the lack of toxicity of the TGFβ3 molecule towards the DK-MG low cell line under the analyzed conditions.

### Example 6. Toxicity analysis of TGFβ1 and known inhibitors of EGFR kinase against fibroblasts using BioStation CT device.

### Day 1

1. Rinse fibroblasts at confluence up to 90% with PBS w/o Mg²⁺ and Ca²⁺. Remove PBS.
2. Detach cells from the culture vessel by adding Trypsin-EDTA (0.05%, 1x) and incubate for 1-3 min at 37°C.
3. Transfer cells to 15 ml conical tube, centrifuge (200xg, 4 min) and suspend in DMEM cell culture medium supplemented with 1% fetal bovine serum and antibiotics.
4. Count cells.
5. Add 5×10⁴ cells per well of 6-well culture plate for adherent culture.
6. Supply with aforementioned cell culture medium and leave for about 12 hours at 37°C.

### Day 2

1. Change culture medium by adding 2 ml of DMEM with antibiotics and 1% serum. Leave for 24 hours.
2. Discard old culture media, add 2 ml per well of fresh medium with antibiotics, w/ or w/o serum and with proper compounds used individually or in combinations (DMSO; maximum volume of tested compounds), erlotinib (1-25 µM), gefitinib (1- 0 µM), afatinib (1-25 µM), TGFβ1 (1-25 ng/ml cat. no. GF111 Merck EMD Millipore) and EGF (20 ng/ml final concentration).
3. Define cell culture plate and each well. Place cell culture vessel with cells into BioStation CT.
4. Perform automatic microscopic observation at 3-hour intervals, for min.100 hours. BioStation CT software allows automatic cell counting and assessment of surface area occupied by these cells. Results should be analysed by CL-Quant Software.

**Result:** The presented result indicates the lack of cytotoxicity of the TGFβ1 molecule on normal cells under the analyzed conditions (Fig. 10). In the case of fibroblasts, even a beneficial effect on the proliferation of these cells was observed.

### Example 7. Toxicity analysis of TGFβ2 and known inhibitors of EGFR kinase against human induced neural stem cells using BioStation CT device.

### Day 1

1. Rinse iNS cells (Neural Induction Stem Cells) at confluence up to 90% with PBS w/o Mg²⁺ and Ca²⁺. Remove PBS.
2. Detach cells from the culture vessel by using StemProAccutase (ThermoFisherScientific, Cat. No. A1110501) for 5 minutes.
3. Transfer cells to 15 ml conical tube, centrifuge (200xg, 4 min) and suspend in PSC Neural Induction Medium (ThermoFisherScentific, Cat. No. A1647801) supplemented with antibiotics.
4. Count cells.
5. Add 1×10⁵ cells per well of 6-well culture plate for adherent culture.
6. Supply with aforementioned cell culture medium and leave for about 12 hours at 37°C.

### Day 2

1. Rinse 2-3 times with PBS w/o Mg²⁺ and Ca²⁺. Remove PBS precisely each time.
2. Add 1.5 ml of PSC Neural Induction Medium with antibiotics and proper compounds used individually or in combinations (DMSO; maximum volume of tested compounds) alone or in combination with EGF (20 ng/ml (Sigma, Cat. no. E9644)), erlotynib (10 µM Molecula; Cat. No. 89983631), TGFβ2 (1; 2,5 and 5 ng/ml; Cat. No SRP3170-5UG; Sigma-Aldrich).
3. Define cell culture plate and each well. Place cell culture vessel with cells into BioStation CT.
4. Perform automatic microscopic observation at 6-hour intervals.

### The following days

1. Change culture media each 24 hours. Do not forget to add DMSO/EGF/erlotinib/TGFβ2 individually or in combinations to proper culture vessels.
2. Place cell culture vessels into BioStation CT.
3. Perform automatic microscopic observation at 6-hour intervals. BioStation CT software allows automatic cell counting and assessment of surface area occupied by these cells.
4. Analogous observations, however, with less accuracy can be performed using JULI microscope or an ordinary light microscope. However, it should be emphasized that BioStation CT enables the preservation of environmental conditions during the experiment as well as the observation of many vessels at the same time.

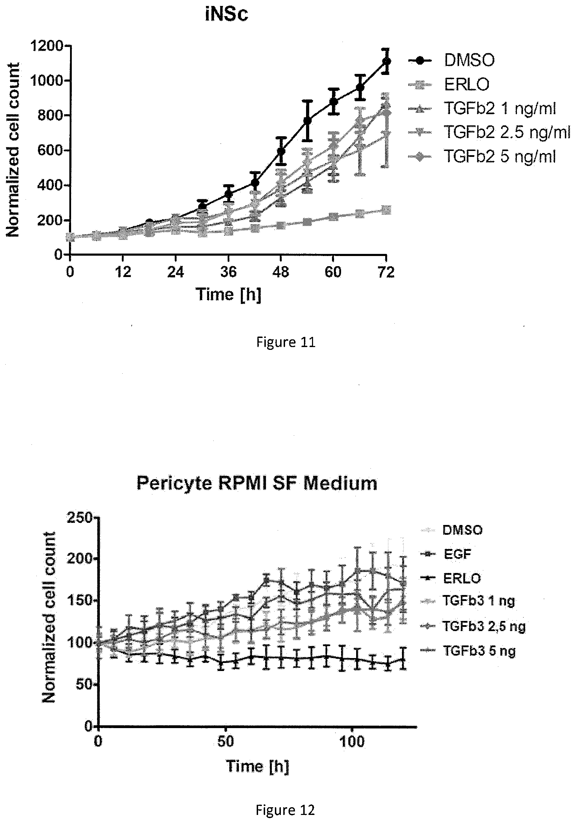

**Results:** Results suggest lack of cytotoxicity of TGFβ2 on human induced neural stem cells at tested conditions in a statistically significant way (Fig. 11). IC-50 value of TGFP2 on used cells was unmeasurable and was over several hundred ng/ml.

### Example 8. Toxicity analysis of TGFβ3 and known inhibitors of EGFR kinase against human normal pericytes using BioStation CT device.

### Day 1

1. Rinse pericytes at confluence up to 90% with PBS w/o Mg²⁺ and Ca²⁺. Remove PBS.
2. Detach cells from the culture vessel by adding Trypsin-EDTA (0.05%, 1x) and incubate for 1-3 min at 37°C.
3. Transfer cells to 15 ml conical tube, centrifuge (200xg, 4 min) and suspend in RPMI 1640 cell medium supplemented with antibiotics.
4. Count cells.
5. Add 4×10⁴ cells per well of 6-well culture plate for adherent culture.
6. Supply with RPMI 1640 cell culture medium and leave for about 24 hours at 37°C.

### Day 2

1. Rinse 2-3 times with PBS w/o Mg²⁺ i Ca²⁺. Remove PBS precisely each time.
2. Add 2 ml of RPMI 1640 culture medium with antibiotics and w/o serum and leave for about 12 hours at 37°C.

### Day 3

1. Rinse cells 2-3 times with PBS w/o Mg²⁺ i Ca²⁺. Remove PBS precisely each time.
2. Add 2 ml of RPMI 1640 culture medium with antibiotics and w/o serum.
3. Do not forget to add (DMSO; max. volume of tested compounds); alone or in combination with EGF (20 ng/ml (Sigma, Cat. no. E9644)), erlotynib (10 µM Molecula; Cat. No. 89983631), TGFβ3 (1; 2,5 i 5 ng/ml Cat. No. PHG9305; Gibco^{™}; Thermo Fisher Scientific). Place cell culture vessels into BioStation CT.
4. Perform automatic microscopic observation at 6-hour intervals. BioStation CT software allows automatic cell counting and assessment of surface area occupied by these cells.

### The following days

1. Change culture media each 48 hours. Do not forget to add DMSO/EGF/erlotinib/TGFβ3 individually or in combinations to proper culture vessels.
2. Place cell culture vessels into BioStation CT.
3. Perform automatic microscopic observation at 3-hour intervals. BioStation CT software allows automatic cell counting and assessment of surface area occupied by these cells.
4. Analogous observations, however, with less accuracy can be performed using JULI microscope or an ordinary light microscope. However, it should be emphasized that BioStation CT enables the preservation of environmental conditions during the experiment as well as the observation of many vessels at the same time.

**Results:** TGFβ3 shows lack of cytotoxicity against normal human pericytes expressing under the tested conditions in a statistically significant way (Fig. 12). IC-50 value of TGFβ3 on this cells were unmeasurable and were more than several hundred ng/ml.

### Example 9. Study of the effect of TGFβ1 on U87-MG cell line

### Day 1

1. Rinse U87-MG cells at confluence up to 90% with PBS w/o Mg²⁺ and Ca²⁺. Remove PBS.
2. Detach cells from the culture vessel by adding Trypsin-EDTA (0.05%, 1x) and incubate for 1-3 min at 37°C.
3. Transfer cells to 15 ml conical tube, centrifuge (200xg, 4 min) and suspend in DMEM cell medium supplemented with antibiotics and 1% fetal bovine serum.
4. Count cells.
5. Add 5×10⁴ cells per well of 6-well culture plate for adherent culture.
6. Supply with DMEM cell culture medium and leave for about 24 hours at 37°C.

### Day 2

1. Change culture medium (DMEM) with antibiotics and w/o serum and leave for about 24 hours at 37°C.
2. Add 2 ml of DMEM culture medium with antibiotics and w/o serum.
3. Do not forget to add tested compounds alone or in combinations (DMSO; max. volume of tested compounds); EGF (20 ng/ml), erlotinib (1-25 µM), gefitinib (1- 0 µM), afatinib (1-25 µM), TGFβ1 (1; 2,5 and 5 ng/ml; Cat. No. GF111 Merck EMD Millipore).
4. Place cell culture vessels into BioStation CT.
5. Perform automatic microscopic observation at 6-hour intervals for minimum 100 hours. BioStation CT software allows automatic cell counting and assessment of surface area occupied by these cells.

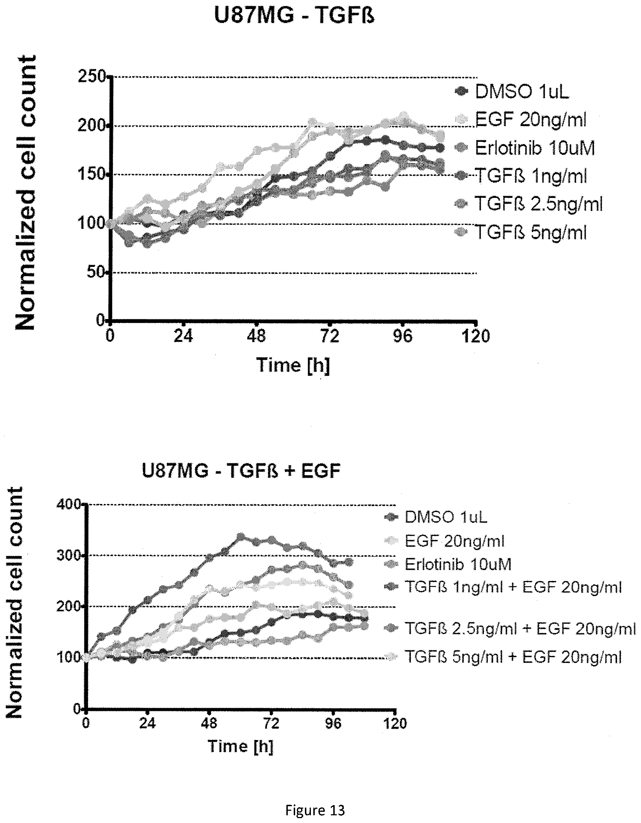

**Results:** Obtained results show the Lack of cytotoxicity of TGFβ1 molecule against U87-MG cells under the tested conditions (Fig. 13). In the case of U87-MG cells even a beneficial effect on the proliferation level of these cells was observed.

### Example 10. Research in an animal model.

NOD.CB17-Prkdcscid / J mice were provided by Animalab (Poland). 4-week-old mice were subjected to a 1-week quarantine. Human glioblastoma cells DK-MG high were cultured in RPMI-1640 medium (Biowest, France) supplemented with 10% fetal bovine serum (PAA, The Cell Culture Company, Austria), penicillin/streptomycin (Life Technologies, USA), gentamicin (Biowest)), FungizoneAntimycotic (Life Technologies), Primocin and Normocin (Invivogen, San Diego, California, USA) at 37°C and 5% CO2 content. Before the start of the experiment, the cells were tested for the contamination with bacteria from the Mycoplasma family.

In NOD.CB17-Prkdcscid / J mice, subcutaneous tumors were implemented in a previously described manner [Fridman 2012]. Briefly, monolayer cells were harvested with trypsin-EDTA (Life Technologies) and washed with PBS. 2 × 10⁶ cells were mixed in equal volumes with Matrigel (BD Biosciences, Belgium) and the mixture was injected subcutaneously into the right and left flanks of the animal. Observations were made daily, tumors were measured once a week as described previously [Ayers 2010]. In short, the two longest axes perpendicular to each other (in the x / y plane) were measured with an electronic caliper and the tumor volume was calculated according to the formula: 0.5xy2. Appearing xenografts were allowed to develop subcutaneously for about 12 weeks due to the growth kinetics of tumors composed of DK-MG high subline cells. After this time, Erlotinib was dissolved in 10% DMSO / 0.45% Methylcellulose (Sigma, USA) and TGFβ1 (catalog number GF111 Merck EMD Millipore) in 0.9% saline was injected twice weekly at a concentration of 50 mg / kg mice (Erlotinib) and 1 mg / kg mice (TGFβ1). In addition, mice bearing DK-MG tumors were injected intratumorally with vehicle (10% DMSO / 0.45% methylcellulose) to establish a negative control for erlotinib testing.

At the end of the experiment, mice were sacrificed according to institutional guidelines. The tumors were extracted for further analysis and organs were separated and checked for possible metastases or damages as a result of treatment.

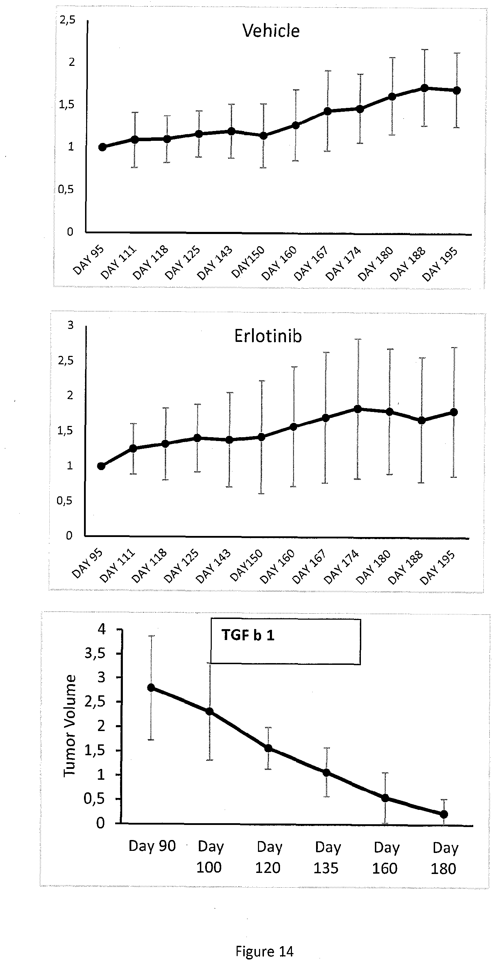

**Results:** Animal studies have demonstrated the efficacy of TGFβ1 against DK-MG tumor cells and its lack of effect on the general condition of animals. There is also a lack of efficacy of erlotinib against DK-MG high cell line and its side effects (Fig. 14).

### Literature:

Stec WJ, Rosiak K, Siejka P, Peciak J, Popeda M, Banaszczyk M, Pawlowska R, Treda C, Hulas-Bigoszewska K, Piaskowski S, Stoczynska-Fidelus E, Rieske P. Cell line with endogenous EGFRvIII expressionis a suitable model for research and drug development purposes. Oncotarget. 2016 May 31;7(22):31907-25. doi: 10.18632/oncotarget.8201
Fridman R, Benton G, Aranoutova, Kleinman HK, Bonfil RD, Increased initiation and growth of tumor cell lines, cancer stem cells and biopsy material in mice using basement membrane matrix protein (Cultrex or Matrigel) co-injection.NatProtoc. 2012 May 17;7(6):1138-44.
Ayers GD, McKinley ET, Zhao P, Fritz JM, Metry RE, Deal BC, Adlerz KM, Coffey RJ, Manning HC, Volume of preclinical xenograft tumors is more accurately assessed by ultrasound imaging than manual caliper measurements. J Ultrasound Med. 2010 Jun;29(6):891-901.

The invention is further illustrated in the preferred examples, with reference to the accompanying illustrations:
**Figure 1**. TGFβ1 structure.
**Figure 2****.** TGFβ2 structure.
**Figure 3****.** TGFβ3 structure.
**Figure 4****.** Results of real-time observation of DK-MG high and low subline cells using BioStation CT. The molecule designated TGFβ1 is anticancer against DK-MG high cells already at a concentration of 1 ng/ml - it inhibits their proliferation and causes cells death. The effect of TGFβ1 is statistically significant.
**Figure 5****.** Results of real-time observation of DK-MG line cells using the BioStation CT device. TGFβ1 is effective against cancer cells of DK-MG high at a concentration of 1 ng/ml - it inhibits their proliferation and causes death despite the use of EGF, which acts pro-proliferatively and anti-apoptotically on DK-MG high subline cells. The effect of TGFβ1 was statistically significant.
**Figure 6****.** Results of real-time observation of DK-MG high subline cells using the BioStation CT device. The molecule designated TGFβ2 is effective against cancer cells of DK-MG high at a concentration of 1 ng/ml - it inhibits their proliferation and causes death. The effect of TGFβ2 is significant.
**Figure 7****.** Results of real-time observation of DK-MG low subline cells using the BioStation CT device. The molecule designated TGFβ2 is ineffective against cancer DK-MG cells at a concentration of 1 ng/ml.
**Figure 8****.** Results of real-time observation of DK-MG high subline cells using the BioStation CT device. The molecule designated TGFβ3 is anticancer-effective against DK-MG high cells already at a concentration of 1 ng/ml - it inhibits their proliferation and causes cells death. The effect of TGFP3 is significant.
**Figure 9****.** Results of real-time observation of DK-MG low subline cells using the BioStation CT device. The molecule designated TGFP3 is ineffective against cancer DK-MG cells at a concentration of 1 ng/ml.
**Figure 10****.** Results of real-time observation of DK-MG low subline cells using the BioStation CT device. The molecule designated TGFβ3 is ineffective against DK-MG low cells at a concentration of 1 ng/ml.
**Figure 11****.** Example of TGFβ2 cytotoxicity analysis on induced normal neural stem cells using BioStation CT. The effect of TGFβ2 is statistically insignificant.
**Figure 12****.** Example of analysis of TGFβ3 cytotoxicity on normal human pericytes using BioStation CT. The effect of TGFP3 is statistically insignificant.
**Figure 13****.** Results of real-time observation of U87-MG cell lines using the BioStation CT device. TGFβ1 is ineffective against U87MG cells at the concentrations tested it does not inhibit their proliferation.
**Figure 14****.** The graphs show the change in the size (volume) of tumors that developed in SCID mice. Observations were made for about 200 days. In animal studies, the efficacy of TGFβ1 has been demonstrated and there is no implied effect on the general condition of animals. There was also a lack of efficacy of erlotinib in the experimental conditions used. The effect of TGFβ1 was statistically significant.

## Claims

1. An active form of transforming growth factor TGFβ1 or TGFβ2 or TGFβ3 for use in the treatment of patients with neoplastic lesions, for which at least part of the cancer cells is EGFRvIII-positive.

2. The active form of transforming growth factor TGFβ1 or TGFβ2 or TGFβ3 for use in the treatment according to claim 1, **characterized in that** EGFRvIII-negative cancer cells are biologically dependent on EGFRvIII-positive cells.

3. The active form of transforming growth factor TGFβ1 or TGFβ2 or TGFβ3 for use in the treatment according to claim 1, **characterized in that** the cancer cell susceptible to its cytotoxic and cytostatic action is EGFRvIII-positive cancer stem cell or EGFRvIII-negative cancer cells are biologically dependent on EGFRvIII-positive cells.

4. The active form of transforming growth factor TGFβ1 or TGFβ2 or TGFβ3 for use in the treatment according to claim 1, **characterized in that** the cancer cells are human cancer cells.

5. The active form of transforming growth factor TGFβ1 or TGFβ2 or TGFβ3 for use in the treatment of patients with glioblastoma, for which at least part of the cancer cells is EGFRvIII- positive.

6. The active form of transforming growth factor TGFβ1 or TGFβ2 or TGFβ3 for use in the treatment according to claim 5, **characterized in that** EGFRvIII-negative cancer cells are biologically dependent on EGFRvIII-positive cells.

7. The active form of transforming growth factor TGFβ1 or TGFβ2 or TGFβ3 for use in the treatment according to claim 1, **characterized in that** transforming growth factor is transforming growth factor beta 1 (TGFβ1).

8. The active form of transforming growth factor beta 1 (TGFβ1) for use in the treatment according to claim 7, **characterized in that** the cancer cell susceptible to its cytotoxic and cytostatic action is EGFRvIII-positive cancer stem cell or EGFRvIII-negative cancer cells are biologically dependent on EGFRvIII-positive cells.

9. The active form of transforming growth factor beta 1 (TGFβ1) for use in the treatment according to claim 7, **characterized in that** the cancer cells are human cancer cells, preferably glioblastoma.

10. The active form of transforming growth factor TGFβ1 or TGFβ2 or TGFβ3 for use in the treatment according to claim 1, **characterized in that** transforming growth factor is transforming growth factor beta 2 (TGFβ2).

11. The active form of transforming growth factor beta 2 (TGFβ2) for use in the treatment according to claim 10, **characterized in that** the cancer cell susceptible to its cytotoxic and cytostatic action is EGFRvIII-positive cancer stem cell or EGFRvIII-negative cancer cells are biologically dependent on EGFRvIII-positive cells.

12. The active form of transforming growth factor beta 2 (TGFβ2) for use in the treatment according to claim 10, **characterized in that** the cancer cells are human cancer cells, preferably glioblastoma.

13. The active form of transforming growth factor TGFβ1 or TGFβ2 or TGFβ3 for use in the treatment according to claim 1, **characterized in that** transforming growth factor is transforming growth factor beta 3 (TGFβ3).

14. The active form of transforming growth factor beta 3 (TGFβ3) for use in the treatment according to claim 13, **characterized in that** the cancer cell susceptible to its cytotoxic and cytostatic action is EGFRvIII-positive cancer stem cell or EGFRvIII-negative cancer cells are biologically dependent on EGFRvIII-positive cells.

15. The active form of transforming growth factor beta 3 (TGFβ3) for use in the treatment according to claim 13, **characterized in that** the cancer cells are human cancer cells, preferably glioblastoma.

## Patentansprüche

1. Eine aktive Form des transformierenden Wachstumsfaktors TGFβ1 oder TGFβ2 oder TGFβ3 zur Verwendung bei der Behandlung von Patienten mit neoplastischen Läsionen, bei denen mindestens ein Teil der Krebszellen EGFRvIII-positiv ist.

2. Aktive Form des transformierenden Wachstumsfaktors TGFβ1 oder TGFβ2 oder TGFβ3 zur Verwendung bei der Behandlung nach Anspruch 1, **dadurch gekennzeichnet, dass** EGFRvIII-negative Krebszellen biologisch abhängig von EGFRvIII-positiven Zellen sind.

3. Aktive Form des transformierenden Wachstumsfaktors TGFβ1 oder TGFβ2 oder TGFβ3 zur Verwendung bei der Behandlung nach Anspruch 1, **dadurch gekennzeichnet, dass** die für seine zytotoxische und zytostatische Wirkung empfängliche Krebszelle eine EGFRvIII-positive Krebsstammzelle ist oder EGFRvIII-negative Krebszellen biologisch von EGFRvIII-positiven Zellen abhängig sind.

4. Aktive Form des transformierenden Wachstumsfaktors TGFβ1 oder TGFβ2 oder TGFβ3 zur Verwendung bei der Behandlung nach Anspruch 1, **dadurch gekennzeichnet, dass** die Krebszellen menschliche Krebszellen sind.

5. Die aktive Form des transformierenden Wachstumsfaktors TGFβ1 oder TGFβ2 oder TGFβ3 zur Verwendung bei der Behandlung von Patienten mit Glioblastom, bei denen mindestens ein Teil der Krebszellen EGFRvIII-positiv ist.

6. Aktive Form des transformierenden Wachstumsfaktors TGFβ1 oder TGFβ2 oder TGFβ3 zur Verwendung bei der Behandlung nach Anspruch 5, **dadurch gekennzeichnet, dass** EGFRvIII-negative Krebszellen biologisch abhängig von EGFRvIII-positiven Zellen sind.

7. Aktive Form des transformierenden Wachstumsfaktors TGFβ1 oder TGFβ2 oder TGFβ3 zur Verwendung bei der Behandlung nach Anspruch 1, **dadurch gekennzeichnet, dass** der transformierende Wachstumsfaktor der transformierende Wachstumsfaktor beta 1 (TGFβ1) ist.

8. Aktive Form des transformierenden Wachstumsfaktors beta 1 (TGFβ1) zur Verwendung bei der Behandlung nach Anspruch 7, **dadurch gekennzeichnet, dass** die für seine zytotoxische und zytostatische Wirkung empfängliche Krebszelle eine EGFRvIII-positive Krebsstammzelle ist oder EGFRvIII-negative Krebszellen biologisch von EGFRvIII-positiven Zellen abhängig sind.

9. Aktive Form des transformierenden Wachstumsfaktors beta 1 (TGFβ1) zur Verwendung bei der Behandlung nach Anspruch 7, **dadurch gekennzeichnet, dass** die Krebszellen menschliche Krebszellen sind, vorzugsweise Glioblastom.

10. Aktive Form des transformierenden Wachstumsfaktors TGFβ1 oder TGFβ2 oder TGFβ3 zur Verwendung bei der Behandlung nach Anspruch 1, **dadurch gekennzeichnet, dass** der transformierende Wachstumsfaktor der transformierende Wachstumsfaktor Beta 2 (TGFβ2) ist.

11. Aktive Form des transformierenden Wachstumsfaktors beta 2 (TGFβ2) zur Verwendung bei der Behandlung nach Anspruch 10, **dadurch gekennzeichnet, dass** die für seine zytotoxische und zytostatische Wirkung empfängliche Krebszelle eine EGFRvIII-positive Krebsstammzelle ist oder EGFRvIII-negative Krebszellen biologisch von EGFRvIII-positiven Zellen abhängig sind.

12. Aktive Form des transformierenden Wachstumsfaktors beta 2 (TGFβ2) zur Verwendung bei der Behandlung nach Anspruch 10, **dadurch gekennzeichnet, dass** die Krebszellen menschliche Krebszellen sind, vorzugsweise Glioblastom.

13. Aktive Form des transformierenden Wachstumsfaktors TGFβ1 oder TGFβ2 oder TGFβ3 zur Verwendung bei der Behandlung nach Anspruch 1, **dadurch gekennzeichnet, dass** der transformierende Wachstumsfaktor der transformierende Wachstumsfaktor beta 3 (TGFβ3) ist.

14. Aktive Form des transformierenden Wachstumsfaktors beta 3 (TGFβ3) zur Verwendung bei der Behandlung nach Anspruch 13, **dadurch gekennzeichnet, dass** die für seine zytotoxische und zytostatische Wirkung empfängliche Krebszelle eine EGFRvIII-positive Krebsstammzelle ist oder EGFRvIII-negative Krebszellen biologisch von EGFRvIII-positiven Zellen abhängig sind.

15. Aktive Form des transformierenden Wachstumsfaktors beta 3 (TGFβ3) zur Verwendung bei der Behandlung nach Anspruch 13, **dadurch gekennzeichnet, dass** die Krebszellen menschliche Krebszellen sind, vorzugsweise Glioblastom.

## Revendications

1. Forme active du facteur de croissance transformant TGFβ1 ou TGFβ2 ou TGFβ3 à utiliser dans le traitement de patients présentant des lésions néoplasiques, pour lesquels au moins une partie des cellules cancéreuses est EGFRvIII-positive.

2. Forme active du facteur de croissance transformant TGFβ1 ou TGFβ2 ou TGFβ3 utilisée dans le traitement selon la revendication 1, **caractérisée par le fait que** les cellules cancéreuses EGFRvIII-négatives sont biologiquement dépendantes des cellules EGFRvIII-positives.

3. Forme active du facteur de croissance transformant TGFβ1 ou TGFβ2 ou TGFβ3 utilisée dans le traitement selon la revendication 1, **caractérisée par le fait que** la cellule cancéreuse sensible à son action cytotoxique et cytostatique est une cellule souche cancéreuse EGFRvIII-positive ou que les cellules cancéreuses EGFRvIII-négatives sont biologiquement dépendantes des cellules EGFRvIII-positives.

4. Forme active du facteur de croissance transformant TGFβ1 ou TGFβ2 ou TGFβ3 utilisée dans le traitement selon la revendication 1, **caractérisée par le fait que** les cellules cancéreuses sont des cellules cancéreuses humaines.

5. Forme active du facteur de croissance transformant TGFβ1 ou TGFβ2 ou TGFβ3 pour le traitement des patients atteints de glioblastome, dont au moins une partie des cellules cancéreuses est positive à I'EGFRvIII.

6. Forme active du facteur de croissance transformant TGFβ1 ou TGFβ2 ou TGFβ3 utilisée dans le traitement selon la revendication 5, **caractérisée par le fait que** les cellules cancéreuses EGFRvIII-négatives sont biologiquement dépendantes des cellules EGFRvIII-positives.

7. Forme active du facteur de croissance transformant TGFβ1 ou TGFβ2 ou TGFβ3 à utiliser dans le traitement selon la revendication 1, **caractérisée par le fait que** le facteur de croissance transformant est le facteur de croissance transformant bêta 1 (TGFβ1).

8. Forme active du facteur de croissance transformant bêta 1 (TGFβ1) utilisée dans le traitement selon la revendication 7, **caractérisée par le fait que** la cellule cancéreuse sensible à son action cytotoxique et cytostatique est une cellule souche cancéreuse EGFRvIII-positive ou que les cellules cancéreuses EGFRvIII-négatives sont biologiquement dépendantes des cellules EGFRvIII-positives.

9. Forme active du facteur de croissance transformant bêta 1 (TGFβ1) pour utilisation dans le traitement selon la revendication 7, **caractérisée par le fait que** les cellules cancéreuses sont des cellules cancéreuses humaines, de préférence des glioblastomes.

10. Forme active du facteur de croissance transformant TGFβ1 ou TGFβ2 ou TGFβ3 à utiliser dans le traitement selon la revendication 1, **caractérisée par le fait que** le facteur de croissance transformant est le facteur de croissance transformant bêta 2 (TGFβ2).

11. Forme active du facteur de croissance transformant bêta 2 (TGFβ2) utilisée dans le traitement selon la revendication 10, **caractérisée par le fait que** la cellule cancéreuse sensible à son action cytotoxique et cytostatique est une cellule souche cancéreuse EGFRvIII-positive ou que les cellules cancéreuses EGFRvIII-négatives sont biologiquement dépendantes des cellules EGFRvIII-positives.

12. Forme active du facteur de croissance transformant bêta 2 (TGFβ2) pour utilisation dans le traitement selon la revendication 10, **caractérisée par le fait que** les cellules cancéreuses sont des cellules cancéreuses humaines, de préférence des glioblastomes.

13. Forme active du facteur de croissance transformant TGFβ1 ou TGFβ2 ou TGFβ3 à utiliser dans le traitement selon la revendication 1, **caractérisée par le fait que** le facteur de croissance transformant est le facteur de croissance transformant bêta 3 (TGFβ3).

14. Forme active du facteur de croissance transformant bêta 3 (TGFβ3) pour utilisation dans le traitement selon la revendication 13, **caractérisée par le fait que** la cellule cancéreuse sensible à son action cytotoxique et cytostatique est une cellule souche cancéreuse EGFRvIII-positive ou que les cellules cancéreuses EGFRvIII-négatives sont biologiquement dépendantes des cellules EGFRvIII-positives.

15. Forme active du facteur de croissance transformant bêta 3 (TGFβ3) pour utilisation dans le traitement selon la revendication 13, **caractérisée par le fait que** les cellules cancéreuses sont des cellules cancéreuses humaines, de préférence des glioblastomes.
